# EUROPEAN PATENT APPLICATION

(11) **EP 4 299 011 A1**
(43) Date of publication of application: **03.01.2024**
(21) Application number: 22193366.6
(22) Date of filing: 01.09.2022
(51) Int. Cl.: A61B 8/08

(54) **PROCESSING A CONTRAST ENHANCED ULTRASOUND IMAGE OF A LESION**

(30) Priority: 30.06.2022 WO PCT/CN2022/102770
(71) Applicant: Koninklijke Philips N.V., 5656 AG Eindhoven (NL)
(72) Inventor: TONG, Ling, Eindhoven (NL); LIN, Qizhong, Eindhoven (NL); CHEN, Zhouye, 5656AG Eindhoven (NL); WANG, Jianan, 5656AG Eindhoven (NL); CHEN, Jinning, Eindhoven (NL); SHIH, Cho-Chiang, Eindhoven (NL); XU, Jingping, Eindhoven (NL); BAI, Xianghui, Eindhoven (NL); ZHOU, Hongyu, Eindhoven (NL); JING, Xiang, Eindhoven (NL)
(74) Representative: Philips Intellectual Property & Standards

(57) **Abstract**

According to an aspect, there is provided a computer implemented method (300) of processing a contrast-enhanced ultrasound image of a lesion. The method comprises comparing (302) properties of a lesion as described in a contrast-enhanced ultrasound report of the contrast-enhanced ultrasound image to a reference in order to determine an inconsistency in the contrast-enhanced ultrasound report; and sending (304) a message to a user device to cause the user device to provide an alert relating to the inconsistency, to provide feedback.

## Description

### FIELD OF THE INVENTION

The disclosure herein relates to processing contrast-enhanced ultrasound images of lesions. More specifically but non-exclusively, embodiments herein relate to providing alerts relating to inconsistencies in contrast-enhanced ultrasound reports.

### BACKGROUND OF THE INVENTION

Liver cancer is one of the leading causes of cancer deaths worldwide. Although both MRI (magnetic resonance imaging) and CT (computed tomography) are recommended medical imaging methods for the diagnosis of liver cancer, both CT and MRI require high cost and complex instrumentation. Contrast-enhanced ultrasound (CEUS) is thus an often-used imaging modality for liver lesion diagnosis due to its low price, fast, and non-invasive imaging.

During the diagnosis of liver tumors using CEUS images, a doctor injects a contrast agent into the blood vessels of a patient. After the injection of contrast agent, clinicians observe and record several CEUS loops as the contrast agent flows through the lesion (or other anatomical feature of interest) during this procedure. Compared with traditional ultrasound modalities, CEUS is more complicated with both spatial and temporal information corresponding to the contrast phases.

During reporting, clinicians write down their observations and features as well as a diagnostic conclusion in a report known as a CEUS report. The Liver Imaging Reporting And Data System (LI-RADS) classification system for liver lesions can help standardize and provide expert consensus on observed features. LI-RADS is described in detail in the paper by Elsayes et al. entitled: "LI-RADS: a conceptual and historical review from its beginning to its recent integration into AASLD clinical practice guidance". J Hepatocell Carcinoma. 2019; 6: 49-69. Published online 2019 Feb 5. doi: 10.2147/JHC.S186239.

### SUMMARY OF THE INVENTION

As noted above, diagnosis of contrast-enhanced ultrasound (CEUS) images is usually made based on the interpretation of the images or loops, e.g. extraction of important features by a doctor. Guidelines such as CEUS LI-RADS which is a classification system for Liver lesions, help provide expert consensus on these features. During reporting, the clinician typically writes down their observations and features before coming to a diagnostic conclusion. Such observations are generally put into a CEUS report.

It is an object of this disclosure to provide improved reporting mechanisms for CEUS examinations. The invention is defined by the independent claims. The dependent claims define advantageous embodiments.

According to a first aspect, there is provided a computer implemented method of processing a contrast-enhanced ultrasound image of a lesion. The method comprises comparing properties of a lesion as described in a contrast-enhanced ultrasound report of the contrast-enhanced ultrasound image to a reference in order to determine an inconsistency in the contrast-enhanced ultrasound report; and sending a message to a user device to cause the user device to provide an alert relating to the inconsistency, to provide feedback.

According to a second aspect, there is an apparatus for processing a contrast-enhanced ultrasound image of a lesion. The apparatus comprises: a memory comprising instruction data representing a set of instructions; and a processor configured to communicate with the memory and to execute the set of instructions. The set of instructions, when executed by the processor, cause the processor to perform the method of the first aspect.

According to a third aspect, there is an ultrasound imaging system comprising the apparatus of the second aspect. The apparatus further comprises a transducer; and the method is performed during a contrast-enhanced ultrasound examination, to provide real-time feedback.

According to a fourth aspect, there is a computer program product comprising a computer readable medium, the computer readable medium having computer readable code embodied therein, the computer readable code being configured such that, on execution by a suitable computer or processor, the computer or processor is caused to perform the method of the first aspect.

Thus, in this manner, inconsistencies in CEUS reports can be determined and alerts can be raised so that a clinician can review such inconsistencies, and either correct the inconsistency or obtain new CEUS images. Thus, an improved CEUS imaging process is provided, improving the quality of CEUS images and their analysis. Improved CEUS imaging and analysis leads to improved patient outcomes.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 is an apparatus according to some embodiments herein;
Fig. 2 is an ultrasound imaging system according to some embodiments herein;
Fig, 3 shows a method according to some embodiments herein; and
Fig. 4 shows an example flow-chart in an ultrasound imaging

### DETAILED DESCRIPTION OF EMBODIMENTS

As noted above, the diagnostic conclusions of a contrast-enhanced ultrasound (CEUS) examination are usually recorded in a CEUS report. Factors such as time-pressure can lead to errors appearing in such reports. For example, there may be inconsistencies in the report, e.g. mutually incompatible information. This can lead to incorrect diagnoses leading to poorer quality care and reduced patient outcomes. The disclosure herein aims to improve report quality in terms of self-consistency and to raise alerts in a manner that can be actioned by a clinician or sonographer so that the inconsistencies can be checked and remedied, and/or so that additional information may be obtained. Three scenarios are mainly considered: 1) consistency of lesion size between B-mode and CEUS; 2) consistency among different but related features; 3) consistency between features and diagnosis conclusion. The disclosure herein is capable of detecting self-inconsistency in these scenarios and ensuring that the clinical user acts accordingly.

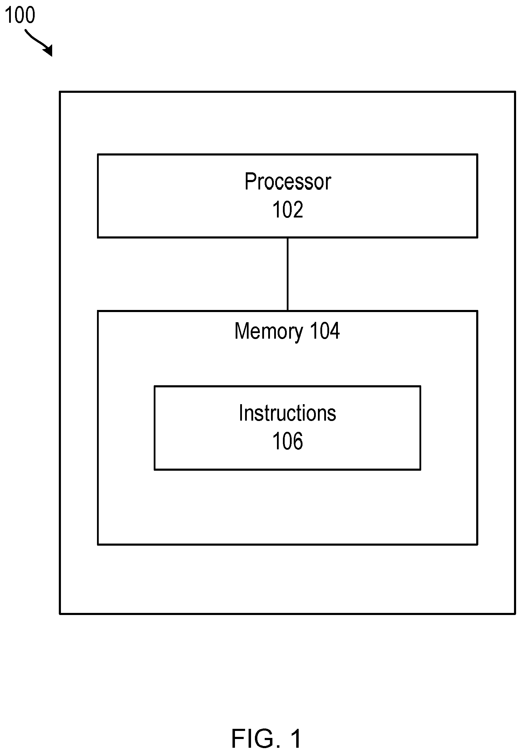

Turning now to Fig. 1, in some embodiments there is an apparatus 100 for use in processing a CEUS image of a lesion. Generally, the apparatus may form part of a computer apparatus or system e.g. a laptop, desktop computer or other computing device. In some embodiments, the apparatus 100 may form part of a distributed computing arrangement or the cloud.

The apparatus comprises a memory 104 comprising instruction data representing a set of instructions 106 and a processor 102 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 100 may be for use in processing a CEUS image of a lesion. More specifically, the set of instructions 106, when executed by the processor, cause the processor to: compare properties of a lesion as described in a CEUS report of the CEUS image to a reference in order to determine an inconsistency in the CEUS report; and send a message to a user device to cause the user device to provide an alert relating to the inconsistency, to provide feedback.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the CEUS images, the CEUS report and/or any other information or data received, calculated or determined by the processor 102 of the apparatus 100 or from any interfaces, memories or devices that are external to the apparatus 100. The processor 102 may be configured to control the memory 104 to store the CEUS images, the CEUS report and/or the any other information or data.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus 100 is incorporated into an ultrasound (US) imaging system. For example, an ultrasound imaging system may comprise the apparatus 100 and a display to display the alert.

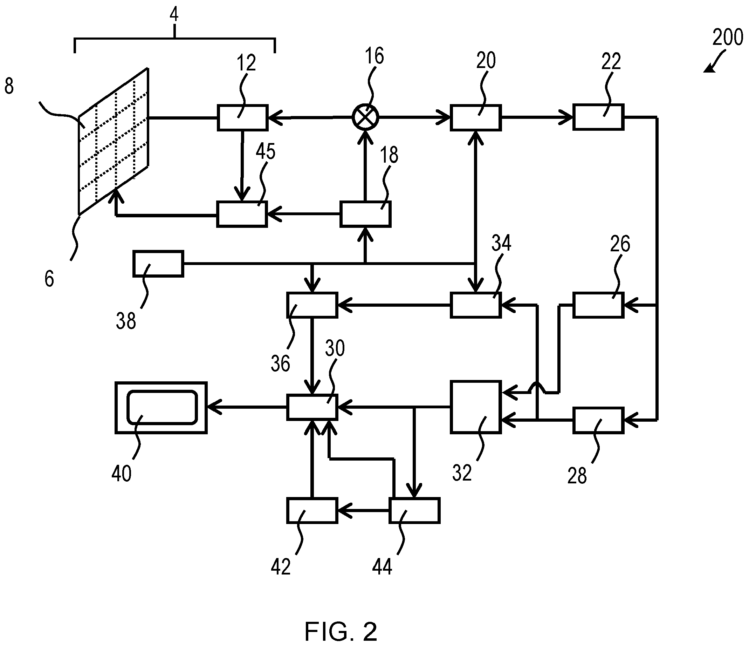

An ultrasound imaging system may further comprise other components, such as those associated with obtaining and processing ultrasound image data. An example ultrasound imaging system 200 is shown in Fig. 2. ultrasound system 200 comprises an array transducer probe 4 which has a transducer array 6 for transmitting ultrasound waves and receiving echo information. The transducer array 6 may comprise CMUT (capacitive micromachined ultrasonic transducers) transducers; piezoelectric transducers, formed of materials such as PZT (lead zirconate titanate) or PVDF (polyvinylidene fluoride); or any other suitable transducer technology. In this example, the transducer array 6 is a two-dimensional array of transducers 8 capable of scanning either a 2D plane or a three-dimensional volume of a region of interest. In another example, the transducer array may be a 1D array.

The transducer array 6 may be coupled to a micro-beamformer 12 which controls reception of signals by the transducer elements. Micro-beamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a micro-beamformer 12, the transducer array may be operated directly by a main system beamformer (not shown in Fig. 2).

The system 200 may further comprise a transmit/receive (T/R) switch 16, which the micro-beamformer 12 can be coupled to and which switches the array between transmission and reception modes. The transmission of ultrasound beams from the transducer array 6 is directed by a transducer controller 18 coupled to the micro-beamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a micro-beamformer) during the transmission mode.

It is noted that in an alternative embodiment, instead of a micro-beamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the micro-beamformer or the main system beamformer depending upon the implementation) activates the transducer array, or a sub-aperture of the transducer array. The sub-aperture may be a one-dimensional line of transducers or a two-dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated, and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above-described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the micro-beamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B-mode (i.e. brightness mode, or 2D imaging mode) processor 26 and a Doppler processor 28. The B-mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B-mode image. The exact location of the pixel within the image is determined by the location of the associated amplitude measurement along the RF signal and the line (beam) number of the RF signal. B-mode images of such structures may be formed in the harmonic or fundamental image mode, or a combination of both as described in US 6,283,919 (Roundhill et al.) and US 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two-dimensional (2D) sector-shaped format, or a pyramidal three-dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US 6,443,896 (Detmer). A volume renderer 42 converts the echo signals of a 3D data set into a projected 3D image as viewed from a given reference point as described in US 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes, the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

It will be appreciated that the ultrasound image system illustrated in Fig 2 is merely an example and that an ultrasound image system may comprise different components to those described above.

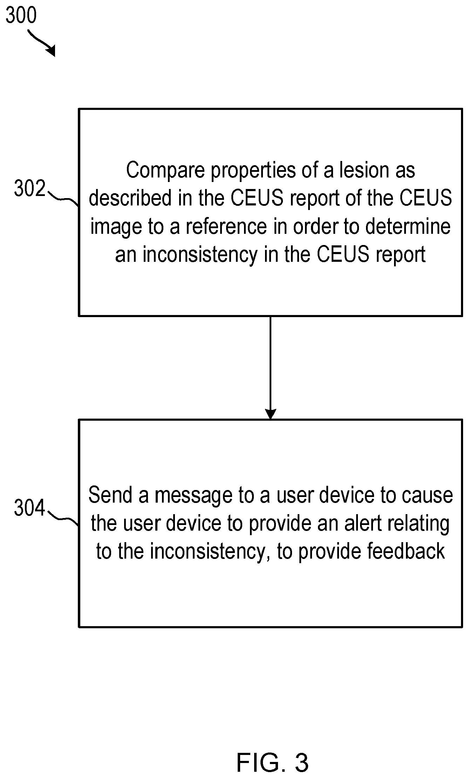

Turning to Fig. 3, there is a computer implemented method 300 for use in processing a contrast-enhanced ultrasound (CEUS) image of a lesion. Embodiments of the method 300 may be performed, for example by an apparatus such as the apparatus 100 or the ultrasound imaging system 200 described above.

Briefly, in a first step 302, the method 300 comprises comparing properties of a lesion as described in a CEUS report of the CEUS image to a reference in order to determine an inconsistency in the CEUS report. In a second step 304 the method comprises sending a message to a user device to cause the user device to provide an alert relating to the inconsistency, to provide feedback.

In more detail, a lesion may refer to any area of abnormal tissue in the body. For example, a lesion may be a tumor, such as a cancerous tumor. Examples of liver tumors include but are not limited to Hepatocellular carcinoma (HCC), Intrahepatic cholangiocarcinoma (bile duct cancer), Angiosarcoma and hemangiosarcoma. The skilled person will appreciate that these are merely examples however and that the lesion may be any type of lesion.

As described above, to obtain CEUS images, a doctor injects a contrast agent into the blood vessels of a patient (intravenous injection). After the injection of contrast agent, clinicians observe and record several CEUS loops as the contrast agent flows through the lesion (or other anatomical feature of interest) during this procedure. Compared with traditional ultrasound modalities, CEUS is more complicated with both spatial and temporal information corresponding to the different contrast phases.

CEUS imaging is described in more detail in the paper by Dietrich et al. entitled: "How to perform Contrast-Enhanced Ultrasound (CEUS)"; Ultrasound Int Open. 2018 Jan; 4(1): E2-E15. Published online 2018 Feb 7. doi: 10.1055/s-0043-123931.

The Dietrich paper notes that CEUS allows real-time recording and evaluation of the wash-in and wash-out phases of the ultrasound contrast agent (UCA) over several minutes. When examining the liver, this provides dynamic visualization of different vascular phases. Owing to the specific supply of blood to the liver three different phases have been defined: the arterial (AP), the portal venous (PVP), and the late (sinusoidal) phases (LP)

A CEUS report is any report that is produced comprising data related to a CEUS image. For example, a CEUS report may comprise images, measurements, observations, notations, or a diagnosis as made by a clinician or sonographer. It is noted that the terms doctor, clinician, sonographer and user may be used interchangeably herein.

A CEUS report may comprise other measurements, such as measurements made or predicted by a diagnostic program. A diagnostic program may refer to a computer aided diagnosis program. A diagnostic program may use machine learning to produce one or more annotations of the CEUS images and these may be recorded in the CEUS report. For example, a machine learning model may be used to annotate a CEUS image with information such as presence/absence of a lesion, position of lesion, size or other dimensions of the lesion.

In some embodiments, segmentation may be used to obtain shape/size information about a lesion in a CEUS image. The skilled person will be familiar with methods of segmentation, for example, model-based segmentation as described in the paper by Ecabert, O., et al. 2008 entitled "Automatic Model-Based Segmentation of the Heart in CT Images"; IEEE Trans. Med. Imaging 27 (9), 1189-1201. Another segmentation method uses machine learning (ML) models to convert an image into a plurality of constituent shapes (e.g. block shapes or block volumes), based on similar pixel/voxel values and image gradients. This is described, for example, in the paper by Long et al. entitled: "Fully Convolutional Networks for Semantic Segmentation".

A CEUS report may be in any format, such as a Microsoft^{™} WORD document, Portable Document Format (pdf) document, Microsoft^{™} powerpoint document, Microsoft^{™} Excel document (xlxs), Text File (txt), Extensible Markup Language (XML) format, Hypertext Markup Language (HTML), Rich Text Format (RTF), OpenDocument Text Document (ODT), JPEG image (JPG), Portable Network Graphic image (PNG) or any other document format.

In step 302, as noted above, the method 300 comprises comparing properties of a lesion as described in a CEUS report of the CEUS image to a reference in order to determine an inconsistency in the CEUS report.

In some embodiments, the properties of the lesion comprise contrast phase information from the CEUS report. The reference may then comprise predefined relationships between different contrast phases according to contrast phase perfusion physiology, and an inconsistency may be determined if the contrast phase information from the CEUS report is inconsistent with the predefined relationships. For example, some phases occur in a particular order, reflecting different stages as the contrast agent flows through the lesion. Thus, the predefined relationships may indicate a sequence in which the different contrast phases should be recorded in the report.

Thus, in some embodiments, an inconsistency is determined if contrast phases are recorded out of chronological sequence in the CEUS report, or in a physiologically implausible combination.

Features in the report can be separated into three groups: time, degree of enhancement (DoE) and pattern of enhancement. The group of time features includes arterial phase (AP) onset time, peak time, hyper enhancing onset time, iso enhancing onset time and hypo enhancing onset time. The group of degree of enhancement includes AP DoE, portal venous phase (PVP) DoE, Late Phase (LP) DoE, early washout and degree of washout. These two groups are related with each other as the features in the second groups are all time dependent. For example, AP DoE represents the DoE of AP, where AP is defined from AP onset time to about 45s. Thus, inconsistency may be determined if the time features and the DoE are recorded in a combination that is physiologically impossible or contrary to the manner in which the respective features are defined. Put another way, an inconsistency may be determined if the time features and DoE are recorded in a manner that is contrary to the underlying relationships between said time features and DoE.

Some example predefined relationships are as follows:
1) The arterial phase (AP) onset time shall always be earlier than the peak time due to the contrast agent's perfusion physiology, thus if one documents the AP onset time larger than the peak time, there is self-inconsistency.
2) If the degree of enhancement (DoE) of a lesion during AP is iso-enhancing, the lesion shall only present as iso-enhancement or hypo-enhancement during the PVP and/or LP given that the contrast agents will not be re-perfused during the PVP and LP, thus if one documents AP DoE as iso-enhancement and PVP DoE as hyper-enhancement and/or LP DoE as hyper-enhancement, there is a self-inconsistency.
3) If the PVP DoE of a lesion is iso-enhancement, then the LP DoE shall only be iso-enhancement or hypo-enhancement due to tissue perfusion physiology, thus if one documents the LP DoE as hyper-enhancement, then there is a self-inconsistency.
4) The degree of enhancement (DoE) of PVP DoE of a lesion is mainly decided by the frames around 120s (from introduction of the contrast agent). That is, if the lesion is iso-enhancing in these frames, then PVP DoE is iso-enhancing. The feature of PVP DoE is related to the degree of washout at 120s. If PVP DoE is iso-enhancing, the degree of washout must be 'none' as the lesion has not washed out yet. Thus, if one documents degree of washout as either "marked" or "mild", there is self-inconsistency.
5) If a lesion presents "early washout", this means the DoE of the lesion before 60 seconds shows hypo-enhancement, hence the PVP DoE and LP DoE shall only be hypo-enhancement, and degree of washout shall not be "none". Thus, if one documents a lesion as "early washout" and the PVP DoE and/or LP DoE is not hypo-enhancement, and/or degree of washout is "none", then there is self-inconsistency.

Put another way, the predefined relationships between different contrast phases may indicate that: AP onset should be before the peak contrast; if the DoE of the lesion during the AP is iso-enhancing, then the lesion should present as iso-enhanced or hypo-enhanced during the PVP and/or LP; if the PVP DoE of a lesion is iso-enhanced, then the LP DoE should be iso-enhancement or hypo-enhancement; if PVP DoE is iso-enhanced, the degree of washout should be none; and/or if the lesion presents early washout then the PVP DoE and LP DoE should only be hypo-enhancement, and degree of washout should be greater than none (e.g. non-zero).

It will be appreciated that these are merely examples and that other properties, and other predefined relationships may equally be used. In general, any inconsistency that may be contrary to the contrast agent's perfusion physiology may be considered.

In one example embodiment, step 302 may be implemented according to the following pseudo-code:

```
 self_consistency = true;
                if aponset time >= peak_time
                          self_consistency = false
                if ap_doe == iso_enhancement & (pvp_doe == hyper_enhancement || lp_doe
 ==hyper_enhancement)
                          self_consistency = false
                if pvp_doe==iso_enhancement & lp doe == hyper_enhancement
                          self_consistency = false
                if pvp_doe==iso_enhancement & washout_degree == none
                          self_consistency = false
                if early_washout == true & washout_degree == none
                          self_consistency = false
                if other_non_logic_feature_statement
                          self_consistency = false
```

Turning now to other embodiments, in some embodiments, a size consistency check is performed. In a CEUS examination, lesion size is often measured more than once, for example, in a B-mode image and in a CEUS image. There is a risk that the lesion size is not consistent in these two measurements, especially when there is more than 1 lesion present in an image. Thus, if the lesion size is different in a CEUS image to that as measured in a B-mode image, and the difference is beyond a certain threshold, then an inconsistency in lesion size may be highlighted.

Thus, in some embodiments, in step 302 the reference comprises properties of the lesion as measured in a second ultrasound image obtained with a different modality to the CEUS image. The properties of the lesion as described in the CEUS report are geometric properties of the lesion as measured in the CEUS image and the references are values of the same geometric properties as measured in the second ultrasound image. An inconsistency may then be determined if the values of the geometric properties of the lesion as measured in the CEUS image are different to the geometric properties of the lesion as measured in the second ultrasound image, by more than a threshold difference. As noted above, the second ultrasound image may be a B-mode ultrasound image.

The geometric properties may comprise a size, radius, diameter, area or circumference of the lesion. These are merely examples however, and any geometric or shape property may be measured between the CEUS image and the second ultrasound image. The threshold difference may be set empirically, e.g. based on a distribution of relative sizes between CEUS images and respective images of the same type as the second image as measured for a representative population.

In this way, discrepancies in measurements between the CEUS image and the second image may be highlighted.

Turning now to other embodiments, in some embodiments, a diagnosis or other derived quantity may be checked in the CEUS report. For example, fields (e.g. features or parameter values) in the CEUS report may be checked for consistency with a diagnosis as found in a medical guideline.

Examples of medical guidelines include but are not limited to those described in the following papers: Chen, M., Yan, K., Dai, Y., Wu, W., Xu, H., Yang, W., ... Yu, X. (2013). "Liver contrast-enhanced ultrasound application guidelines" (China, updated 2012). Chin. J. Ultrasonogr., 22(8), 696-722; Claudon, M., Dietrich, C. F., Choi, B. I., Cosgrove, D. O., Kudo, M., Nolsøe, C. P., ... Chaubal, N. G. (2013). Guidelines and good clinical practice recommendations for contrast enhanced ultrasound (CEUS) in the liver-update 2012. Ultraschall in der Medizin-European Journal of Ultrasound, 34(01), 11-29; Kono, Y., Lyshchik, A., Cosgrove, D., Dietrich, C. F., Jang, H. J., Kim, T. K., ... Kambadakone, A. (2017). Contrast enhanced ultrasound (CEUS) liver imaging reporting and data system (LI-RADS®): the official version by the American College of radiology (ACR). Ultraschall in der Medizin-European Journal of Ultrasound, 38(01), 85-86. The skilled person will appreciate that these are merely examples however and that other medical guidelines may equally be used.

For example, the medical guidelines may provide (e.g. indicate or suggest) a diagnosis based on a given set of feature values (being present or observed). If the clinician has come to a diagnosis result that is not suggested or consistent with the medical guidelines for a given set of parameter values in the CEUS report, then this may be highlighted as an inconsistency. The information in the CEUS report may thus be compared to a medical guideline in order to check for mutual consistency.

Generally, the reference properties may be obtained from the medical guideline. In some embodiments, user input may be received indicating a particular medical guideline that is to be used. In other words, a user may indicate a guideline that is to be used for the consistency check. In such embodiments, the reference properties may be obtained from the indicated medical guideline.

The reference properties may be associated with a diagnosis in the CEUS report. The inconsistency may thus be determined if the properties in the CEUS report are incompatible with the reference properties in the medical guideline, for the diagnosis in the CEUS report. Thus, in this way, the method 300 may be used to determine whether the properties in the CEUS report are inconsistent with the diagnosis in the CEUS report (e.g. by comparison with the medical guideline).

Turning back to Fig. 3, in step 304 the method comprises sending a message to a user device to cause the user device to provide an alert relating to the inconsistency, to provide feedback.

A user device may be, for example, a display (e.g. a computer display), a warning light, or any other device that may be used to provide the alert to the user.

The alert may be any type of alert. For example, a visual alert such as a message on a display, a tactile alert such as a vibration of a user input device (e.g. mouse or similar), or an audio alert such as a beep, or computer generated audio message. The alert may highlight that there is an inconsistency. The alert may indicate one or more portions of the CEUS report that are inconsistent. The alert may indicate that the CEUS scan needs to be repeated.

In another example, when the apparatus detects an inconsistency in lesion size, or features, or diagnosis, the apparatus may ask the user to review and/or confirm the inconsistent fields in the CEUS report.

In some embodiments, the method 300 is performed in real time, for example during a CEUS examination, and/or whilst the sonographer is creating the CEUS report. In such examples, the alert may suggest in real time that a new CEUS scan is performed due to the inconsistency. In this way, the method 300 may be used in real time to indicate to the sonographer that further scans are likely to be needed, and to prompt the sonographer to obtain the necessary information in order to make an accurate diagnosis, thus improving the diagnosis, leading to improved patient care and improved patient outcomes.

Feedback may comprise, for example, an indication of the inconsistency, an indication that a measurement should be repeated, an indication that one or more fields in the report should be reviewed, that scans may need to be repeated, or any other feedback to the user.

It will be appreciated that the alerts indicated above are merely examples and that other alerts and other types of feedback are equally possible as a result of the method 300.

The method 300 may be triggered in various ways, for example, in some embodiments, after the CEUS examination, a report generation User Interface (UI) may be shown to the user. This may be initiated by the user, e.g. the user may confirm that they want to go to the reporting step. Features that are related to diagnosis may be shown in the UI. In embodiments where the user selects a guideline against which to form the comparison in step 302, the UI may give the user a list of guidelines so that they can choose the guideline they would like to refer to/use in the self-consistency check in terms of diagnosis.

Generally, the method 300 may be triggered, for example, when the clinical user confirms that they have finished providing manual input. Thus, the apparatus may use the method 300 to check the CEUS report consistency.

Thus, there is provided a CEUS reporting system with an automated quality check in terms of self-consistency in feature interpretation, diagnosis and lesion size. Said CEUS reporting system is able to provide the alert to a user to alert when self-inconsistency is detected.

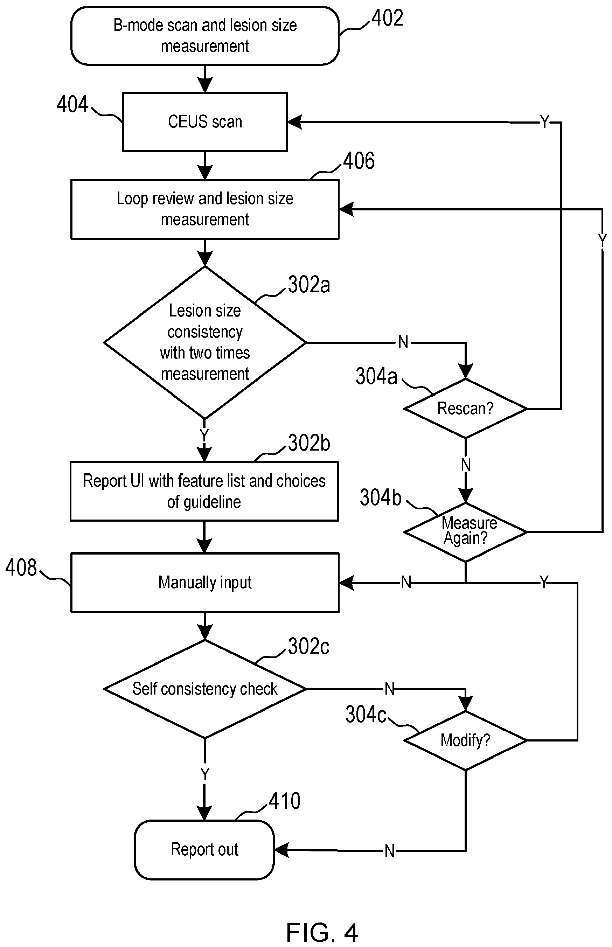

Turning now to Fig. 4 which shows an example embodiment of the method 400 according to some embodiments herein. In this embodiment, in step 402, a B-mode scan of the lesion is made, from which a lesion size measurement is made. In step 404 a CEUS scan is made. In step 406, the size of the lesion is measured in the CEUS image(s).

The method 300 is then performed on the measured sizes. In step 302a, a consistency check is performed between the CEUS measurement and the B-mode measurement (according to step 302 as described above). If the two measurements are not within a threshold difference of each other, then in step 304a, the method comprises sending a message to a user device to cause the user device to provide an alert relating to the inconsistency (according to step 304 described above). In step 304a, the alert asks the user whether a rescan might be necessary, if the user declines, then a second alert is generated (according to step 304) in step 304b which suggests that the lesion measurements should be repeated.

The method 300 is then repeated to determine whether there is an inconsistency in the diagnosis recorded in the CEUS report. In step 302b, the user is presented with different guidelines to choose from for the comparison. In step 408, the user provides an indication of the guideline to be used. In step 302c, properties of the lesion as described in the CEUS report of the CEUS image are compared to the selected medical guideline (which is used as a reference) in order to determine an inconsistency in the CEUS report (according to step 302 described above).

If an inconsistency is determined, then an alert is raised in step 304c (as described with respect to step 304 above), asking the user whether any of the inconsistent fields should be modified. If not, then the report is output in step 410.

Turning now to other embodiments, in another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. Measures recited in mutually different dependent claims may be advantageously combined. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method (300) of processing a contrast-enhanced ultrasound image of a lesion, the method comprising:
comparing (302) properties of the lesion as described in a contrast-enhanced ultrasound report of the contrast-enhanced ultrasound image to a reference in order to determine an inconsistency in the contrast-enhanced ultrasound report; and
sending (304) a message to a user device to cause the user device to provide an alert relating to the inconsistency, to provide feedback.

2. A method (300) as claimed in claim 1, wherein
the properties of the lesion comprise contrast phase information from the contrast-enhanced ultrasound report;
the reference comprises predefined relationships between different contrast phases according to contrast phase perfusion physiology; and
an inconsistency is determined if the contrast phase information from the contrast-enhanced ultrasound report is inconsistent with the predefined relationships.

3. A method (300) as claimed in claim 2, wherein an inconsistency is determined if contrast phases are recorded out of chronological sequence in the contrast-enhanced ultrasound report, or in a physiologically implausible combination.

4. A method (300) as claimed in claim 2 or 3, wherein the predefined relationships between different contrast phases indicate that:
arterial phase onset should be before the peak contrast;
if the degree of enhancement of the lesion during the arterial phase is iso-enhancing, then the lesion should present as iso-enhanced or hypo-enhanced during the portal venous phase and/or late phase;
if the portal venous phase degree of enhancement of a lesion is iso-enhanced, then the late phase degree of enhancement should be iso-enhancement or hypo-enhancement;
if portal venous phase degree of enhancement is iso-enhanced, the degree of washout should be none; and/or
if the lesion presents early washout then the portal venous phase degree of enhancement and late phase should only be hypo-enhancement, and degree of washout should be greater than none.

5. A method (300) as claimed in any one of the preceding claims, wherein the reference comprises properties of the lesion as measured in a second ultrasound image obtained with a different modality to the contrast-enhanced ultrasound image.

6. A method (300) as claimed in claim 5, wherein the properties of the lesion as described in the contrast-enhanced ultrasound report are geometric properties of the lesion as measured in the contrast-enhanced ultrasound image and the references are values of the same geometric properties as measured in the second ultrasound image; and
wherein the inconsistency is determined if the values of the geometric properties of the lesion as measured in the contrast-enhanced ultrasound image are different to the geometric properties of the lesion as measured in the second ultrasound image by more than a threshold difference.

7. A method (300) as claimed in claim 6, wherein the geometric properties comprise a size, radius, diameter, area or circumference of the lesion.

8. A method (300) as claimed in any one of the preceding claims, wherein the reference comprises a medical guideline and the method further comprises:
obtaining reference properties from the medical guideline wherein the reference properties are associated with a diagnosis in the contrast-enhanced ultrasound report; and
wherein the inconsistency is determined if the properties in the contrast-enhanced ultrasound report are incompatible with the reference properties in the medical guideline, for the diagnosis in the contrast-enhanced ultrasound report.

9. A method as claimed in claim 8, wherein the alert indicates that the properties in the contrast-enhanced ultrasound report are inconsistent with the diagnosis in the contrast-enhanced ultrasound report.

10. A method (300) as claimed in claim 8 or 9, further comprising:
receiving user input indicating a particular medical guideline that is to be used;
and wherein the reference properties are obtained from the indicated medical guideline.

11. A method (300) as claimed in any one of the preceding claims, wherein the method is performed during a contrast-enhanced ultrasound examination, to provide real-time feedback.

12. A method (300) as claimed in claim 11, wherein the alert suggests in real time that a new contrast-enhanced ultrasound scan is performed due to the inconsistency.

13. An apparatus (100) for processing a contrast-enhanced ultrasound image of a lesion, the apparatus comprising:
a memory (104) comprising instruction data representing a set of instructions (106); and
a processor (102) configured to communicate with the memory and to execute the set of instructions, wherein the set of instructions, when executed by the processor, cause the processor to carry out the method of any one of claims 1 to 12.

14. An ultrasound imaging system (200) comprising the apparatus (100) of claim 13, and a transducer (6).

15. A computer program product comprising computer readable code, the computer readable code being configured such that, on execution by a suitable computer or processor, the computer or processor is caused to perform the method as claimed in any one of claims 1 to 12.
